# EUROPÄISCHE PATENTSCHRIFT

(11) **EP 0 754 453 B1**
(45) Veröffentlichungstag und Bekanntmachung des Hinweises auf die Patenterteilung: **16.01.2002**
(21) Anmeldenummer: 96111437.8
(22) Anmeldetag: 16.07.1996
(51) Int. Cl.: A61K 31/165, A61K 31/245, A61K 31/445, A61K 9/70

(54) **Lokalanästhetika zur topischen Therapie von Kopfschmerzen**
Local anesthetics for topical treatment of headache
Application topique d'anesthétiques locaux pour traiter les maux de tête

(30) Priorität: 17.07.1995 DE 19526019
(43) Veröffentlichungstag der Anmeldung: 22.01.1997
(73) Patentinhaber: EpiCept Corporation, Englewood Cliffs, N.J. 07632 (US)
(72) Erfinder: Liedtke, Rainer K., Dr., 82027 Grünwald (DE)
(74) Vertreter: Tiedtke, Harro, Dipl.-Ing.

(56) Entgegenhaltungen:
- US-A- 5 008 289
- US-A- 5 411 738
- US-A- 5 415 866

## Beschreibung

Die Erfindung betrifft eine Verwendung zur topischen Therapie der Symptomatiken von Kopfschmerzen, einschließlich der Migräne.

Es ist bekannt, daß die attackenweise auftretende Kopfschmerz Symtomatik überwiegend auf neurologischen und vasculären Ursachen beruht, wobei u.a. psychosomatische Stressfaktoren, physikalische Umweltfaktoren, belastungsabhängige funktionelle oder organische Wirbelsäulenstörungen, Reizung bestimmter Hirnnerven, oder auch ein Abusus von Stoffen wie Alkohol, Nikotin sowie auch von Analgetika selbst involviert sein kann.

Diese Symptomatiken werden pharmakologisch überwiegend mit systemisch wirksamen, nichtopioiden, oralen oder injektablen, Analgetika und Antiphlogistika behandelt, partiell in Kombination mit psyschosomatischen oder physikalisch Behandlungen, teilweise auch mit anderen Methoden wie z.B. der Akupunktur.

Die Pharmakotherapien stellen aber insgesamt noch keine ausreichend verträgliche und effektive Behandlungsformen dar. Als pharmakologisches Prinzip werden insbesondere Derivate der Salicysäure, vorzugsweise Acetylsaliclsäure, nichtsteroidale Antiphlogisika, z.B. Ibuprofen, oder Anilin-Derivate, z.B. Paracetamol, eingesetzt (e.g. Brune K. Beck W. in: M. Zenz, I. Jura (Edits.) Lehrbuch der Schmerztherapie, WFG, Stuttgart 1993, S.121-135). Bei Migräne erfolgt zusätzlich der Einsatz von Ergotamin-Derivaten, verschiedentlich Tranquillantien der Benzodiazepin-Gruppe, sowie zur Prophylaxe ein Einsatz von Betablockern.

Allen systemischen Kopfschmerz-Therapien gemeinsam ist eine erhebliche Nebenwirkungsquote. Bei Salyclsäure-Derivaten und nichtsteroidalen Antiphlogistika finden sich insbesondere erhebliche Magenstörungen, als Folge des antiproliferativen Wirkungsmechanismus. Bei Paracetamol findet sich eine metabolische Belastung von Leber- und Nieren-Funktionen, insbesondere bei längerem wie höher dosiertem Gebrauch. Bei Ergotaminderivaten findet sich insbesondere eine sehr hohe Rate an gastrointestinalen Übelkeitsgefühlen. Die Anwendung dieser systemischen Therapien wird somit durch das jeweilige Spektrum der produktspezifischen unerwünschten Wirkungen limitiert, da sich systemische Interventionen alle Organe und Organsysteme einbeziehen.

Als ein besser wirksames pharmakologisches Prinzip wäre eine geeignete Form der Anwendung niedrig dosierter Lokalalänästhetika zu betrachten. Amid- und Ester-Lokalanästhetika, z.B. das Lidocain vom Amid-Typ, zeigen als pharmakologischen Wirkungsmechanismus eine Hemmung des schnellen Natrium Ionen-Influx in Nervenfasern. Sie blockieren hierüber die Impulsleitung der Nervenbahn, was prinzipiell alle regionalen Nervenfasern betrifft. Die sensorischen und anatomisch dünnernen Fasern sind infolge ihrer Morphologie empfindlicher als die motorischen Fasern (Strichartz, G.R. (Ed.) Local Anesthetics, Handbook of Experimental Pharmacology, Vol. 81, Springer, Berlin - New York, 1987). Hieraus lassen sich auch Wirkungseffekte differenzieren.

Eine systemische Anwendung von Lokalanästhetika wäre invasiv, mittels Injektionen möglich. Dies entfällt aber schon weitgehend auf Grund einer Gefahr systemischer Überdosierung mit u.a. ernsten kardialen Nebenwirkungen. Direkte Anwendung von Lokalananästhetika durch lokale Injektionen am Kopf ist technisch möglich und wird auch verschiedentlich ausgeführt. Doch sind lokale Injektionen nicht nur schmerzhaft, sie können auch von den Patienten in keinem Fall selbst ausgeführt werden. Die lokal oberflächliche Injektionstechnik beruht auf der sogenannten Neuraltherapie mit Triggerpunkten (J.T. Travell, D.G. Simons, Myofascial Pain and Dysfunction, Vol I/II, Williams & Wilkins, Baltimore, 1983) und setzt eine erfahrene ärzliche Handhabung und Technik voraus. Sie ist daher auf den Einsatz von klinisch schwerwiegenderen Störungen begrenzt. Der Einsatz konventioneller topischer Formulierungen, z.B. Cremes, erlaubt u.a. weder exakte Dosierung noch Positionierung, wie auch keine kontinuierliche Penetration über längere Anwendungsdauer.

Die US-A-5 411 738 beschreibt Behandlungen zur Reduzierung von mit Nervenverletzungen verbundenen Schmerzen, die durch Herpes zoster oder post-herpetischer Neuralgia verursacht wurden, indem eine lokale Betäubung mittels topischer Anwendung von Lidocain an der Stelle des Schmerzes induziert wird. Als mögliche topische Zubereitungen kommen ein Gel, welches mit einer okklusiven Abdeckung versehen sein kann, oder ein Pflaster, in welchem Lidocain mit einem Klebstoff eingebracht werden kann, in Frage. Je nach der anatomischen Stelle des durch Herpes-zoster-Befall oder den durch post-herpetische Neuralgia veursachten Neuropathien wird die Lidocain-haltige und ggf. trägergebundene Zubereitung im Thorax-Bereich (für post-herpetische Neuralgia) oder im Bereich von Stirn, Schläfe und Kopfhaut (bei Trigemini-Nerv-assoziierten Neuralgia-Zuständen) angewandt. In weiteren experimentellen Studien werden die Behandlung von schmerzhaft empfindlichen Hautzuständen (Alodynia) mittels Anwendung auf die betroffenen Bereiche des Torsos oder der Gliedmaßen beschrieben.

Die US-A-5 415 866 offenbart ein spezielles topisches System, um eine lokale Hautbetäubung zum Schutz vor kleineren chirurgischen Haut-Eingriffen zu erreichen. Dabei können auch Lokalanästhetika zum Einsatz kommen.

Die US-A-5 008 289 betrifft Zusammensetzungen zur Behandlung von Kopfschmerzen. Die Zusammensetzung enthält als wirksames Mittel Capsaicin sowie ein Lokalanästhetikum. Capsaicin ist somit das eigentliche, gegen Kopfschmerzen eingesetzte Mittel, während die Lokalanästhetika wie Lidocain und Benzocain lediglich zur Minderung des durch Capsaicin selbst erzeugten, anfänglichen Schmerzes dient. Die Zusammensetzung ist zum Einträufeln in die Nase ausgelegt.

Der Erfindung liegt die Aufgabe zugrunde, die Therapie der Symptomatik von Kopfschmerzen zu verbessern.

Diese Aufgabe wird gelöst durch Verwendung eines Lokalanästhetikums zur Herstellung einer Zusammensetzung für eine topische Therapie des Kopfschmerzes, wobei die Lokalanästhetikum-enthaltende Zusammensetzung in ein für die intakte Frontal- und Schläfen-Haut des Kopfes geeignetes, topisches Trägersystem mit einer therapeutischen Dosis des Lokalanästhetikums eigebracht ist und wobei das topische Trägersystem in der Lage ist, das Lokalanästhetikum gezielt auf die unter dem topischen Trägersystem liegende Hautregion aufzubringen.

Um die Therapie zu erweitern, ist in einer weiteren Ausbildung der Erfindung, als weiteres therapeutisches Anwendungsgebiet für ein topisches Trägersystem mit Lokalanästhetika auch die Migräne einbezogen.

Um Wirksamkeit und Verträglichkeit der topischen Therapie zu verbessern sind, in einer weiteren Ausbildung der Erfindung Lokalanästhetika vom Amid- oder Ester-Typ enthalten, insbesondere Lidocain, Tetracain, Bupivacain, Prilocain, Mepivacain, Etidocain, sowie Procain und Benzocain, wobei diese Stoffe in Konzentrationsbereichen von 0,5%-40% vorliegen.

Um Wirksamkeit und Verträglichkeit der Therapie zu verbessern werden, in einer weiteren Ausbildung der Erfindung, in dem eingesetzten topischen Trägersytem 2 Lokalanästhetika mit unterschiedlicher Pharmakokinetik kombiniert, wobei diese Einzelstoffe in solchen Konzentrationen vorliegen, daß die Gesamkonzentration beider Wirkstoffe nicht mehr als 40% beträgt.

Um die Therapie insgesamt sicherer und besser handhabbar zu machen, liegt, in einer weiteren Ausbildung der Erfindung, das topische Trägersystem in solchen Formen vor, die den speziellen Gegebenheiten des Appikationsfeldes Stirn- und Schläfenhaut des Kopfes entsprechen, und die äußere Form des topischen Trägersystems rund, oval, eckig, mit konkaven oder konvexen Aussenformen ist, oder das Trägersystem auch mit oder ohne zusätzliche Hilfsmittel vom Anwender in entsprechende Formen segmentiert werden kann.

Eine Therapie der Kopfschmerz Symptomatik mittels eines auf Fronttal- oder Schläfenhaut des Kopfes applizierten Trägersystems mit Lokalanästhetika wurde bisher weder vorgenommen noch beschrieben. Als ein mit der Erfindung erzielter Vorteil ergibt sich somit, daß mit diesem neuen Prinzip erstmals auch eine nichtinvasive und lokale Behandlungsmöglichkeit von Symptomatiken des Kopfschmerzes gegeben ist.

Die topische Therapie mit Lokalanästhetika in einem Trägersystem ermöglicht eine lokal gezielte und dauerhafte therapeutische Beeinflussung terminal und funktionell vernetzter Nervenbahnen im Kopfbereich.

Da dieser Bereich auch über gute cutane Resorptionskapazität verfügt, kann zudem mit nur geringen topischen Dosierungen vorgegangen werden. Systemische Gefährdung, wie bei üblich oralen oder injektablen Analgetika und Antiphlogisika gegeben, wird vermieden, da die Lokalanästhetika, z.B. Lidocain, bei der retardierten cutanen Aufnahme weit überwiegend metabolisiert werden, so daß auch keine systemischen Wirkspiegel mit den entsrechenden Organbelastungen auftreten.

Die Therapie ist über geringe Dosen steuerbar und längeranhaltend und kann nach Bedarf durch Abnahme des Trägers unterbrochen werden.

Insgesamt weist die topische Therapie der Kopfschmerzen somit nicht die systemischen Nebeneffekte wie systemische analgetische oder antiphlogistische Therapien, da eine vorherige Wirksstoff-Distribution über den gesamten Körper vermieden wird, die die anderen Organe und Organsysteme belastet. Zudem verfügen Lokalanästethika auch über eine gute lokale Gewebeverträglichkeit.

Als Beispiele für technisch geeignete Ausgestaltung eines topischen Trägersystems für die Frontal-und Schläfenhaut des Kopfes mit Lokalanästhetika wird auf die Beschreibungen der technischen Trägersysteme US-No.4,765,986, EP 0205974, DE P3716575.5-45 und DE P3811564. 6-45 verwiesen, jedoch ohne es damit auf diese beschriebenen Techniken beschränken zu wollen.

Zur topischen Therapie von Symptomatiken des Kopfschmerzes werden für die Frontal- und Schläfen-Haut des Kopfes geeignete, topische Trägersysteme mit einer therapeutischen Dosis an Lokalanästhetika eingesetzt.

Die Anwendung der Therapieform bezieht dabei insbesondere und als weitere medizinische Indikatione auch eine topische Behandlung der Migräne ein.

Die topische Therapie mit einem Trägersystem mit Lokalanästhetika stellt eine neue nichtinasive, nebenwirkungsarme und effektive, Behandlungsmöglichkeit für die Symptomatik von Kopfschmerzen einschließlich der Migräne dar.

## Patentansprüche

1. Verwendung eines Lokalanästhetikums zur Herstellung einer Zusammensetzung für eine topische Therapie des Kopfschmerzes, wobei die Lokalanästhetikum-enthaltende Zusammensetzung in ein für die intakte Frontal- und Schläfen-Haut des Kopfes geeignetes, topisches Trägersystem mit einer therapeutischen Dosis des Lokalanästhetikums eingebracht ist und wobei das topische Trägersystem in der Lage ist, das Lokalanästhetikum gezielt auf die unter dem topischen Trägersystem liegende Hautregion aufzubringen.

2. Verwendung nach Anspruch 1, **dadurch gekennzeichnet, dass** als weiteres neues medizinisches Anwendungsgebiet für ein topisches Trägersystem mit Lokalanästhetika auch die Migräne einbezogen ist.

3. Verwendung nach vorhergehenden Ansprüchen, **dadurch gekennzeichnet, dass** in dem topischen Trägersystem Lokalanästhetika vom Amid- oder Estertyp eingesetzt werden, insbesondere Lidocain, Tetracain, Prilocain, Bupivacain, Mepivacain, Etidocain sowie Procain und Benzocain, wobei diese Stoffe in Konzentrationsbereichen von 0,5%-40% vorliegen.

4. Verwendung nach vorhergehenden Ansprüchen, **dadurch gekennzeichnet, dass** in dem topischen Trägersystem zwei Lokalanästhetika mit unterschiedlicher Pharmakokinetik kombiniert sind, wobei diese Einzelstoffe in solchen Konzentrationen vorliegen, dass die Gesamtkonzentration beider Wirkstoffe nicht mehr also 40% beträgt.

## Claims

1. Use of a local anaesthetic for the production of a composition for a topical therapy for headache, wherein the composition containing the local anaesthetic is incorporated into a topical carrier system suitable for the intact frontal and temporal skin of the head with a therapeutic dose of the local anaesthetic, and wherein the topical carrier system is capable of selectively applying the local anaesthetic to the skin region under the topical carrier system.

2. Use according to Claim 1, **characterized in that** migraine is included as a further medical application for a topical carrier system with local anaesthetics.

3. Use according to preceding claims, **characterized in that** local anaesthetics of the amide or ester type are used in the topical carrier system, in particular lidocaine, tetracaine, prilocaine, bupivacaine, mepivacaine, etidocaine, and procaine and benzocaine, these substances being present in concentrations of 0.5% - 40%.

4. Use according to preceding claims, **characterized in that** two local anaesthetics with different pharmacokinetics are combined in the topical carrier system, wherein these individual substances are present in concentrations such that the total concentration of both substances is not more than 40%.

## Revendications

1. Utilisation d'un anesthésique local pour la préparation d'une composition destinée au traitement topique du mal de tête, la composition contenant l'anesthésique local étant introduite dans un système de support topique convenable pour la peau intacte de la tête sur le front ou les tempes, à une dose thérapeutique de l'anesthésique local, et le système de support topique étant en mesure d'appliquer de manière ciblée l'anesthésique local sur la région de la peau se trouvant au-dessous du système de support topique.

2. Utilisation selon la revendication 1, **caractérisée en ce qu'**un autre domaine d'application médical pour un système de support topique contenant des anesthésiques locaux est la migraine.

3. Utilisation selon les revendications précédentes, **caractérisée en ce que** l'on utilise dans le système de support topique des anesthésiques locaux du type amide ou ester, en particulier la lidocaïne, la tétracaïne, la prilocaïne, la bupivacaïne, la mépivacaïne, l'étidocaïne, ainsi que la procaïne et la benzocaïne, ces substances se trouvant à des concentrations comprises entre 0,5 et 40 %.

4. Utilisation selon les revendications précédentes, **caractérisée en ce que** l'on combine dans le système de support topique deux anesthésiques locaux ayant une pharmacocinétique différente, ces substances individuelles se trouvant à des concentrations telles que la concentration totale des deux substances actives n'est pas supérieure à 40 %.
